# EUROPÄISCHE PATENTANMELDUNG

(11) **EP 1 972 691 A1**
(43) Veröffentlichungstag der Anmeldung: **24.09.2008**
(21) Anmeldenummer: 08004440.7
(22) Anmeldetag: 11.03.2008
(51) Int. Cl.: C12P 5/02, C12M 1/113

(54) **Verfahren zur Herstellung von Biogas durch diskontinuierliche, 2-stufige Trockenfermentation**

(30) Priorität: 17.03.2007 DE 102007012861
(71) Anmelder: Mineralit GmbH, 18299 Laage / Kronskamp (DE)
(72) Erfinder: Augustin, Winfried, Dipl.-Ing. (FH), 17207 Röbel / Müritz (DE)

(57) **Zusammenfassung**

Gegenstand ist ein Verfahren zur Erzeugung von Biogas durch diskontinuierliche Trockenfermentation nachwachsender Rohstoffe (NaWaRo), vorzugsweise Maissilage sowie von Gras-, Frischgetreide- oderSonnenblumensilage und organischen Rückständen, Nebenprodukten und Abfällen aus Haus- und Landwirtschaft mit vorrangig stichfester Konsistenz in einer Fermenteranlage bei gleichzeitigem Eintrag von Prozesswärme und dem Besprühen der frischen Biomasse mit Methanbaklerien angereicherter Impfkultur.

Dazu wird das frische Biomaterial zu einem Substrat zerkleinert und anschließend in einem geschlossenen sauerstofffreien, auf eine mesophile oder thermophile Prozesstemperatur von 36 bis 38°C oder 50 bis 57°C erwärmten Mischbehälter (4) eingesetzt, mit mesophilen oder thermophilen Bakterien angereichertem Impfkonzentrat besprüht, gemischt und in einer Verweilzeit von 6 bis 10 Tagen zu einer durchgreifenden Gärung gebracht. Dann wird das Substrat aus dem Mischbehälter (4) abgezogen und in einen geschlossenen und auf die Prozesstemperatur von 36 bis 38°C oder 50 bis 57°C erwärmten Gärbehälter (14) umgesetzt, wiederholt umgewälzt und bei einer Verweilzeit von ca. 25 Tagen zur Abschlussvergärung gebracht. Die Gärreste werden aus dem Gärbehälter (14) ausgebracht und ausgepresst. Die ausgepresste Gärflüssigkeit und ein kleiner Teil von Substrat (ca. 5%) aus dem Mischbehälter (4) wird in einen Impfkulturtank (8) abgeleitet, der auf eine Prozesstemperatur von 36 bis 38°C oder 50 bis 57°C erwärmt ist. Hier wird die Gärflüssigkeit mit Methanbakterien angereichert und zum Besprühen des frischen Biomaterials auf Vorrat gehalten.

Dass bei der Fermentation erzeugte Biogas wird in einem BHKW (23) verströmt und zu Wärme umgeformt. Zugleich wird die vom Biogas und den Gärresten mittransportierte Wärmeenergie und die beim Auspressen anfallende Aggregatwärme abgegriffen und maßgeblich als Prozessenergie genutzt. Dazu bestehen der Mischbehälter (4), die Gärbehälter (14), der Impfkulturtank (8) und der Aufnahmetrichter (18) des Pressschneckenseparators (17) aus hochfesten, korrosiv beständigen Mineralgussplatten (5) mit integrieten Heizrohrregistern (5.2) (s. Fig.1).

## Beschreibung

Gegenstand der Erfindung ist ein Verfahren und Einrichtung zur Biogaserzeugung durch diskontinuierliche Trockenfermentation nachwachsender Rohstoffe (NaWaRo), organischen Rückständen, Nebenprodukten und Abfällen aus Haus- und Landwirtschaft mit vorrangig stichfester Konsistenz.

Verfahren und Anlagen zur Gewinnung erneuerbarer Energie auf der Grundlage von Biogas kommen in immer größerem Umfang zum Einsatz. Besonders im landwirtschaftlichen Bereich werden Biogasanlagen eingesetzt, da hier das größte Potential nachwachsender Rohstoffe als Energieträger in Form von Feststoffsubstrat verfügbar ist.

Die Biogaserzeugung erfolgt auf dem Wege der Nass- und Trockenfermentation unter Sauerstoffabschluss durch bakterielle Umsetzung des Substrates zu 2/3 Methan (CH₄) und einem Drittel Kohlendioxid (CO₂) Der Sauerstoffabschluss ist notwendig, da Methanbakterien strikt Anerobier sind, d. h. sie sind nur in einer sauerstofffreien Atmosphäre lebensfähig. Nach einer Entschwefelung des erzeugten Biogases erfolgt die Verstromung und Wärmegewinnung durch Verbrennen in einem Blockheizkraftwerk (BHKW).

Ein großer Anteil der verfügbaren Biomasse wird nach dem Verfahren der Nassfermentation verarbeitet.Voraussetzung bei diesem Verfahren ist, dass der Fermenterinhalt pumpbar und mittels mechanischer, hydraulischer oder pneumatischer Rührsysteme homogenisierbar ist. Als Träger- und Transportflüssigkeit werden daher regelmäßig Gülle und Rezirkulate eingesetzt. Der Einsatz fester strukturreicher Substrate (insbes. NaWaRo) ist nur in begrenztem Umfang möglich.
In der zukünftigen Landwirtschaft wird die Nassfermentation nur auf Bereiche begrenzt sein, wo ausreichend Flüssigkeitsträger (Gülle aus der intensiven Viehwirtschaft) verfügbar ist. Hierbei ist zu beachten, dass Gülle aus der Schweinezucht nur bedingt verwendbar ist, da diese in der Regel mit Antibiotika belastet und biologisch inaktiv ist.

Emeuerbare Energie in der Landwirtschaft wird sich daher vorrangig auf den Einsatz nachwachsender Rohstoffen (NaWaRo), vorrangig Maissilage, konzentrieren.

Entsprechend diesen Bedingungen werden strukturreiche Substrate (NaWaRo), die weder pump- noch fließfähig sind und eine stichfeste Konsistenz aufweisen, maßgeblich durch die Trockenfermentation zur rationellen Produktion von Biogas eingesetzt.

Das Verfahren der Trockenfermentation findet ebenfalls unter Sauerstoffabschluss durch bakterielle Umsetzung des Substrates statt. Zur Sicherung der Lebensfähigkeit der Bakterienkulturen ist eine intensive Befeuchtung notwendig um das Substrat allseitig mit aktiven Methanbakterien zu belegen. Die optimale Substratfeuchte beträgt 60 bis 70%, d. h. der Trockensubstanzgehalt liegt zwischen 20 bis 40%. Zur Befeuchtung und Beimpfung wird vorrangig eine mit Methanbakterien gesättigte Flüssigkeit, das Perkolat, eingesetzt.

Die Prozesstemperatur bei der Trockenfermentation kann in Abhängigkeit der Bakterienkulturen unterschiedlich sein.
Bei psychrophilen Bakterien reicht die Prozesstemperatur bis 25°C. Hier ist ein Aufheizen des Substrates nicht erforderlich, was jedoch zeitlich die Abbauleistung und Gasproduktion verringert.
Thermophile Bakterien benötigen dagegen eine Prozesstemperatur von 50 bis 57°C. Hier ist die Gasausbeute am höchsten, der Energiebedarf zum Aufheizen eines Fermenters ist hier jedoch relativ hoch.

Eine effektive Methanerzeugung wird mit mesophilen Bakterien erreicht, wobei die Prozesstemperatur von 32 bis 42°C reicht und im Optimum bei 38°C liegt.
Beim Einsatz mesophiler Bakterien ist der Energiebedarf relativ niedrig und die Biogasausbeute relativ hoch.

Die erforderliche Wärmeenergie zur Sicherung der Prozesstemperatur und zur Beschleunigung der Fermentation wird erreicht, indem Wärmeenergie durch Beheizen der Fermenterwände und Bodenfläche in das Substrat eingeleitet oder das Perkolat über Wärmeaustauscher erwärmt wird.

Fermenter aus Beton mit integrierten Wärmeübertragern haben den Nachteil, dass der Wärmeübergang unzureichend ist. Das führt u. a. dazu, dass die Steuerung des Fermentierprozesses relativ träge verläuft. Zusätzlich müssen diese Fermenter zusätzlich vor aggressiven Medien während des Fermentationsprozesses geschützt werden.

Ausgehend von dem Fakt, dass der Wärmeübergang bei der Fermentation von Trockenmasse sehr gering ist, wird das Substrat vor der Fermentation und dem Verschließen des Fermenters belüftet. Damit soll Wärmeenergie eingespart werden, indem durch die Belüftung ein Eintrag von Luftsauerstoff erfolgt und so das Einsetzen des aeroben Rotteprozess im Substrat bewirkt und durch Rottewärme die erforderliche Prozesstemperatur erzeugt wird.

Die Umsetzung des Substrates im Fermenter findet in Abhängigkeit der Prozessdauer diskontinuierlich statt. Der Fermenter wird mit Substrat befüllt und verschlossen. Die Biogaserzeugung verläuft dann ohne weitere Substratzugabe, bis das eingebrachte Material vollständig abgebaut ist.

Um eine stabile Methanbakterienpopulation und hohe Bakteriendichte zu erreichen, erfolgt bei einer diskontinuierlichen Fermentation eine Befeuchtung des Substrates mit einer Perkolationsflüssigkeit (bekannt auch als Garagen- bzw. Batch-Verfahren). Auf diesem Wege kann auch die Steuerung der Fermentertemperatur erfolgen.

Die Impfung des Subtrates kann durch ein direktes oder zusätzliches Vermischen des Substrates mit bereits vergorenem Material vorgenommen werden. Bei einigen Substraten beträgt der Anteil an bereits vergorenem Material (Impfmaterial) bis zu 85%. Dadurch kann jedoch sehr wenig frisches Substrat in den Fermenter eingebracht und zu Biogas umgesetzt werden /1/.

Ein Nachteil des Batch-Verfahren besteht auch darin, dass nach jedem Befüllen des Fermenters der Fermentationsprozess neu gestartet werden muss und das sich keine stabile Fermenterbiologie ausbildet.

Die Trockenfermentation kann nach verschiedenen Verfahrensformen erfolgen, die nachstehend kurz genannt werden.

Bei dem Perkolations-Verfahren nach dem System BIOferm® wird die zu vergärende Biomasse gemeinsam mit dem Impfmaterial in den Garagenfermenter einglagert und unter Sauerstoffabschluss vergoren. Die Temperierung und Beimpfung mit CH₄-Bakterien erfolgt über die Rezirkulation des Perkolats.

Beim BEKON®-Trockenfermentationsverfahren wird ständig während des Vergärungsprozesses eine mit Methanbakterien angereicherte Lösung über das Substrat versprüht. Am Boden wird es über ein Drainsystem aufgenommen und in einem Perkolattank zwischengespeichert. Ein Nachteil besteht darin, dass sich im Substrat Sickerkanäle bilden können und dass trockene Nester im Substrat verbleiben, in denen keine Biogasproduktion erfolgt.

Bekannt ist auch die Trocken-Nass-Simultan-Vergärung der LOOCK BIOGAS SYSTEM GmbH. Nach dem Befüllen wird der Fermenter geschlossen und kurzzeitig belüftet bis sich das Substrat durch die einsetzenden aeroben Rotteprozesse erwärmt. Nach Erreichen der Gärtemperatur von ca. 38°C wird die Belüftung gestoppt und das Fermentermaterial mit Perkolat berieselt. Das abgesetzte Perkolat wird danach einem Nassfermeter zugeführt, der zugleich als Speicher dient. Hier wird die organische Fracht des Perkolats zu Biogas abgebaut. Danach wird das Perkolat in einem Wärmeaustauscher wieder auf die Prozesstemperatur erwärmt. Auch bei dieser Methode können sich wie vorgenannt Sickerkanäle mit Trockennestern bilden, die einer vollständigen bakteriellen Umsetzung zu Biogas entgegenwirken bzw. den Umsetzungsprozess erheblich verzögern.

Ein Nachteil bei den vorgenannten Verfahren besteht auch darin, dass vor jeder Entleerung der Fermenter mit Frischluft durchgespült werden muss. Damit wird verhindert, dass die Ausbildung einer dem Menschen schädlichen Atmosphäre (Methangas) und die Ausbildung eines explosionsfähigen Luft-Gas-Gemisches erfolgt. Das auf diese Weise ausgespülte Restgas muß anschließend über einen Biofilter in die Atmosphäre oder thermisch entsorgt werden. Diese Vorgehensweise ist kostenaufwendig und letztlich umweltbelastend.

Für eine kontinuierliche Trockenfermentation sind u. a. nachfolgende Verfahren bekannt.

Bei dem von dem INGENIEURBÜRO RATZKA entwickelten Verfahren wird das zu vergärende Substrat in boxenähnlichen Fermentern, die mit einer Folie verschlossen werden, eingefüllt. Anschließend erfolgt eine Beimpfung durch Überstauen (Überflutung) mit Prozessflüssigkeit, um eine vollständige Durchfeuchtung des Substrates zu erreichen. Zur Sicherung einer kontinuierlichen Biogasproduktion werden mehrere (Trocken-)Fermenter zeitlich versetzt betrieben, die über im Boden und Wandflächen eingebaute Heizelemente beheizt werden. Wie bereits angeführt, ist bei Fermentern aus Beton das Beheizen durch die schlechte Wärme-leitfähigkeit des Material nicht effizient und wenig flexibel, d. h. die Prozesssteuerung ist träge.

Eine Trockenvergärung im Propfenstrom mit liegendem Fermenter ist von der Fa. KOMPO-GAS AG bekannt. Hier wird das Substrat in einen Beschicker eingelagert und per Schneckenförderer in den Fermenter transportiert und dort mit schon vergorenem Material vermischt. Zum Mischen wird ein durchlaufendes Rührwerk mit wechselnder Drehrichtung benutzt. Durch fortlaufendes Einpressen von frischem Substrat durchwandert das eingetragene Substrat propfenartig den Fermenter. Am Ende wird das ausgefaulte Substrat einer Abpressung zugeführt und in eine flüssige Fraktion (Rezirkulat) und eine feste Fraktion (Rohkompost) getrennt. Mit dem Rezirkulat wird das neue Material wieder angemischt. Die Effektivität bei diesem Verfahren ist auf Grund des laufenden Mischens mit schon vergorenem Material relativ gering. Auch ist davon auszugehen, dass durch den propfenartigen Substratvorschub der Verschleiß des Schneckenförderers auf Grund der zu bewegenden Trockenmasse besonders groß und damit wartungsaufwendig ist.

Ein kontinuierliches Trockenfermentierverfahren nach dem Propfenstromprinzip von LIND-LARAN vergärt Biomaterial, das zuvor mit vergorenem Material bzw. Gärrückstand vermischt und danach durch ein Feinkalibrierer gepreßt wird. Dieser Vorgang ist sehr Energieaufwendig und durch die zu kalibrierende Trockensubstanz verschleißbelastend für das Werkzeug. Das kalibrierte Substrat wird in einen liegenden Fermenter gefördert und hier mittels quer liegenden Rührwerken bei periodisch wechselnder Drehrichtung kontinuierlich gemischt. Der Substrattransport durch den Fermenter erfolgt über den Nachschub von frischem Substrat. Das Restsubstrat wird in einem Separator entwässert und das Presswasser als Prozesswasser zum Anmischen des frischen Substrates eingesetzt.

Aus DE 44 23 099 A1 ist ein Verfahren zur Aufbereitung und Vorbehandlung von Bioabfall aus der Landwirtschaft zur Vergärung zu Methan bekannt. Das Vorsortieren, Zerkleinern und Mischen des Bioabfalls erfolgt in einem Zwischenbunker mit nachgelagertem Vorlagebehälter. Von hier erfolgt die Einspeisung in den Fermenter über einen Förderschacht, der zugleich als Wärmetauscher fungiert. Zum Anstoßen der Gärung wird der Bioabfall im Förderschacht mit erwärmtem Presswasser und/oder Restschlamm (Impfgut) vermischt. Im Fermenter erfolgt dann auf Grundlage eines aeroben Gärprozesses die Biogaserzeugung. Am Ende des Gärprozess wird das Faulgut hochgradig entwässert (ausgepresst) und ein Teil davon als Impfgut in den Förderschacht zurückgeführt. Das Presswasser, das Gärbakterien aufweist, wird in einem Bassin gesammelte und im Kreislauf in einem Wärmetauschregister, der mit Warmwasser von 65°C betrieben wird, erwärmt und zur Feuchtigkeitsregulierung im Förderschacht dem frischen Bioabfall zugeführt. Das Beimpfen mit Faulgut und die Feuchtigkeitsregulierung mit erwärmten Presswasser zum Anstoß einer anaerobe Gärung in der Vorbehandlung des Bioabfalls ist nicht optimal, da nachfolgend im Fermenter die erforderliche Prozesstemperatur für eine aeroben Gärung erst über den Aufbau der notwendigen Rottewärme erreichbar ist. Damit ist dieses Verfahren relativ träge.

Mit DE 10 2005 037 452 B3 wurde auch ein Verfahren zur quasi kontinuierlichen Trockenfermentation vorgeschlagen. Dabei werden kleinstrukturierte, zu vergärende Feststoffe (Biomasse) in einer Vorstufe unterhalb eines im Fermenter befindlichen Gärstapels eingetragen, mit einer im Kreis geführten Förderbahn durchmischt und über eine Fußboden-und/oder Wandheizung erwärmt. Zugleich wird die Biomasse mit aus dem Gärstapel über einen verstellbaren Spaltboden niedertropfende Perkolatflüssigkeit und/oder durchfallende kleinen Gärfraktionen versetzt und damit die Gärung angestoßen. Nach kurzen Förderbahnumläufen (ca. 3 Runden (s. 0055)) wird die Biomasse zur vollständigen Gärung in den Fermenter umgesetzt und mit einem Schleppbalken auf dem Gärstapel verteilt.
Zum Volumenausgleich der sich im Fermenter absetzenden Biomasse wird weitere frische Biomasse in den Raum unter dem Spaltboden eingetragen und wie vorgenannt zur Gärung aufbereitet. Nach einer Verweilzeit von ca. 30 Min. (s. 0062) wird diese angemischte Biomasse zur Gärung auf den Gärstapel umgesetzt.
Die Fermenterentleerung erfolgt schichtweise über den Spaltboden in den unterflurigen Raum, wo die vergorene Biomasse nochmals vermischt und erwärmt wird um eingeschlossenes und letztes Biogas bildende Aktivitäten auszulösen. Danach wird die vergorene Biomasse mit Hilfe der Förderbahn ausgetragen.

Dieses Verfahren ist durch die technologische Abfolge in der Vorstufe und die technische Ausstattung sehr aufwendig. Insbesondere der bau- und steuerungstechnische Aufwand und die Wartung der Förderbahn, der Schleppvorrichtung im Fermenter und des verstellbaren Spaltboden ist erheblich. Ebenso die zusätzliche Nachgärung nach dem Abzug vom Gärstabel.

Aus DE 10 2005 029 306 A1 ist weiterhin ein Verfahren zum Betreiben einer Feststofffermenteranlage mit mindestens zwei diskontinuierlich und zeitlich zueinander versetzt betriebenen Feststofffermentern bekannt, wo die eingetragenen Feststoffe im Fermenter mit einem Perkolat durchströmt werden. Das abfließende Perkolat der Fermenter wird in einem beheizten Regenerationsfermenter gesammelt und erneut den Fermentern zugeführt.
Bei dieser Methode können sich in den eingetragenen Feststoffen Sickerkanäle bilden, so dass im Substrat zeitweilig trockene Nester entstehen, in denen keine Biogasproduktion erfolgt bzw. nachhaltig verzögert wird. Der gleiche Effekt kann durch ungleichmäßige Verteilung des Perkolat eintreten, indem es zur partiellen Übersättigung der Feststoffe und damit zu einer Versäuerung kommen.

Ziel und Aufgabe der Erfindung ist es, die Effektivität einer diskontinuierlichen Trockenfermentation zur Erzeugung von Biogas aus nachwachsenden Rohstoffen (NaWaRo), vorzugsweise Maissilage als Basissubstrat sowie von Gras- oder Frischgetreide-, Sonnenblumensilage u. ä. als auch von organischen Rückständen, Nebenprodukten und Abfällen aus Haus- und Landwirtschaft mit vorrangig stichfester Konsistenz zu erhöhen. Insbesondere soll eine vollständige Freisetzung der in der Biomasse gespeicherten Energie auf dem Weg einer Methangärung erreicht werden. Dabei ist zu gewährleisten, dass die Erzeugung von Biogas soweit zu optimieren ist, dass eine kontinuierliche Verstromung und Wärmeerzeugung durch Verbrennen von Biogas in einem Blockheizkraftwerk (BHKW) gewährleistet ist.
Die Methangärung soll sowohl im mesophilen als auch im thermophilen Bereich möglich sein. Ebenso soll die Trockfermentation mit diskontinuierlicher Zuführung von Substrat aus NaWaRo, vorzugsweise Mais-, Gras- oder Frischgetreide-, Sonnenblumensilage als auch organischer Rückstände, Nebenprodukte und Abfälle aus Haus- und Landwirtschaft soll umweltschonend in einem vollklimatisierten Anlagesystem unter Ausschluss von Luftsauerstoff und voll mechanisiert erfolgen. Weiterhin soll zur Aufrechterhaltung einer gleichmäßigen, kontinuierlichen und vollständigen Vergärung der Biomasse eine Impfkultur mit hohem Sättigungsgrad an mesophilen oder thermopilen Bakterien zur Verfügung stehen. Die Entsorgung von Restgas in die Atmosphäre soll nicht erfolgen.

Das entwickelte Verfahren zur diskontinuierlichen Trockenfermentierung gemäß der vorgenannten Zielstellung und die dazu entwickelte Einrichtung sind durch die in Anspruch 1 und Anspruch 8 aufgeführten Merkmale gekennzeichnet.

Das entwickelte Verfahren zur diskontinuierlichen Trockenfermentierung von Biomasse aus nachwachsenden Rohstoffen, vorzugsweise Maissilage als auch aus organischen Rückständen, Nebenprodukten und Abfällen aus Haus- und Landwirtschaft mit vorrangig stichfester Konsistenz umfasst die energetische Umsetzung zu Biogas (Methangas) auf der Grundlage eines zweistufigen sauerstofffreien Verrottungsprozesses unter Einhaltung einer konstanten Prozesstemperatur während der gesamten Phase der Biogaserzeugung im mesophilen oder thermophilen Bereich bei einer durchgreifenden Benetzung der Biomasse mit mit mesophilen oder thermophilen Bakterien angereichertem und gesättigtem Impfkonzentrat.

Dazu erfolgt in einem ersten Schritt die Zerkleinerung der zur Energiegewinnung vorgesehenen Biomasse zu einem gleichmäßigen Substrat mit einer Körnung unter 10mm, vorzugsweise von ca. 3mm. Dieses zerkleinerte Substrat wird mit einem stetig arbeitenden Förderer (Förderschnecke oder Becherwerk (Elevator)) in einen geschlossenen klimatisierten Mischbehälter gefördert, bis dieser zu 2/3 befüllt ist. Die Innentemperatur im Mischbehälter ist beim Einsatz mesophiler Bakterien auf eine Prozesstemperatur von 32 bis 42°C, vorzugsweise 36°C bis 38°C oder bei einem Betrieb mit thermophilen Bakterien auf 50 bis 57 °C erwärmt.

Im zweiten Schritt wird das im Mischbehälter eingebrachte Substrat kontinuierlich umgewälzt, über Boden- und Wandflächen beheizt und zugleich mit einem auf die vorbestimmte Prozesstemperatur erwärmten und mit Methanbakterien angereicherten Impfkonzentrat beimpft, bis eine allseitige Benetzung und Durchfeuchtung des Substrates erreicht ist.
In der Startphase der Anlage werden zur Beimpfung das Impfkonzentrat aus Fremdanlagen entnommen und die erforderliche Wärmeenergie hilfsweise vom Blockheizkraftwerk (BHKW) eingesetzt.

Durch die optimalen klimatischen Lebensbedingungen für die Bakterien, insbesondere durch die allseitige Benetzung und Durchfeuchtung der gleichmäßig zerkleinerten Substratteilchen, wird ein schneller Beginn einer aneroben Gärung und eine aktive Biogasproduktion bewirkt. Dieser Prozess setzt bereits kurz nach Beginn der Hydrolyse - Fett, Eiweiß und Kohlehydrate werden zu Aminosäuren, Zucker und Fettsäure zerlegt - mit steigender Tendenz ein. Das erzeugte Methangas wird aus dem Mischbehälter über einen Kondensatabscheider und Wärmeaustauscher in einen zentralen Gastank abgeleitet, um von dort in einem BHKW verstromt und in Wärme umgesetzt zu werden.

Die Angärphase im Mischbehälter ist nach einer Verweilzeit 8 bis 10 Tagen abgeschlossen. Das Substrat befindet sich dann in einem durchgehend angegorenen Zustand.

Im dritten Schritt wird das beimpfte, auf Prozesstemperatur von ca. 36 bis 38°C bzw. 50 bis 57°C erwärmte Substrat aus dem Mischbehälter in einen abgeschlossenen klimatisierten und auf die Prozesstemperatur von 36 bis 38°C bzw. 50 bis 57°C erwärmten Gärbehälter unter Ausschluss von Sauerstoff umgesetzt.
Parallel wird aus dem Mischbehälter eine kleine Teilmenge von bis zu 5% des angegorenen Substrates zur Erzeugung des Impfkonzentrates in einen Impfkulturtank überführt.

Im Gärbehälter wird unter Aufrechterhaltung einer optimalen Prozesstemperatur von 36 bis 38°C bzw. 50 bis 57°C das eingesetzte Substrat in Abständen von 3 bis 6 Tage langsam umgewälzt und zur restlichen Biogasproduktion gebracht. Mit dem Umwälzen wird eine unerwünschte Gasschichtbildung bzw. ein Verkrusten oder Verkleben des Substrates verhindert und eine gleichmäßige intensive Biogasproduktion erreicht. Das gewonnene Biogas wird aus denn Gärbehälter abgeleitet und über einen Kondensatabscheider und Wärmeaustauscher dem Gastank zugeführt. Nach einer Verweildauer von ca. 25 Tagen ist der Vergärungsprozess abgeschlossen.

Im vierten Schritt wird der Gärrest aus dem Gärbehälter abgezogen und in einem Pressschneckenseparator u. ä. trockengepresst. Der Entzug von Gärflüssigkeit beträgt wenigstens 75%. Die ausgepressten Gärreste werden in ein überdachtes Gärrestelager verbracht bzw. direkt als Kompost an die Verbraucher ausgeliefert. Die ausgepresste Gärflüssigkeit wird zur weiteren Verwendung in den Impfkulturtank abgeleitet. Vor dem Auspressen wird die von den Gärresten mittransportierte und die im Separator anfallende Wärmeenergie abgegriffen. Diese Wärmeenergie wird über einen geschlossenen Kreislauf dem Mischbehälter, Gärbehälter und Impfkulturtank prozessabhängig als Prozesswärme zugeführt.

Analog wird im Kondensabscheider und im Wärmeaustauscher die vom Biogas mittransportierte Wärmeenergie abgegriffen und über den gleichen Kreislauf als Prozessenergie zurückgeführt. Fehlende Wärmeenergie, insbesondere in der Anfahrphase der Trockenfermentation wird hilfsweise im BHKW abgegriffen.

Um eine konstante Biogasproduktion zu gewährleisten, werden mindestens drei Gärbehälter im Parallelbetrieb gefahren. Ihre Anzahl und die Intervalle zur Befüllung der einzelnen Gärbehälter mit vorgegärtem Substrat aus dem Mischbehälter ergibt sich aus dem Verhältnis der Verweilzeit des Substates im Gärbehälter von ca. 25 Tagen und der Vorgärzeit der frisch eingetragenen Biomasse von 8 bis 10 Tagen im Mischbehälter.

Damit wird eine kontinuierliche Biogaserzeugung mit relativ hohem Methananteil bis zur vollständigen Erschöpfung der Methangaserzeugung erreicht und damit ein sicherer, effektiver Betrieb des BHKW gewährleistet. Im BHKW erfolgt, wie bekannt, das Verbrennen des Biogases und damit die Verstromung und die Erzeugung von Wärmeenergie.
Da in Abhängigkeit der eingesetzten Ausgangsstoffe, insbesondere bei der Verwertung organischer Rückstände, Nebenprodukten und Abfällen aus Haus- und Landwirtschaft Schwankungen in der Qualität des erzeugten Biogases mit unterschiedlich zündfähigem Methangehalt auftreten können, ist zur Verbrennung des Biogases im BHKW ein Zündstrahlmotor vorgesehen. Im Zündstrahlmotor kann Methangas ab einer Konzentration von 27 Vol.% unproblematisch verbrannt werden.

Alternativ zum BHKW oder auch im Verbund kann die Verbrennung des Gases in einer stationären Wirbelschichtfeuerung mit nachfolgender Abgaswärmenutzung effektiv erfolgen. Eine stationäre Wirbelschichtfeuerung bietet den Vorteil, dass hier Methangas mit einer Methangaskonzentrationen unter 27 Vol.% (Schwachgas) unproblematisch verbrannt werden kann.

Im geschlossenen und auf Prozesswärme klimatisierten Impfkulturtank werden die gesammelte Gärflüssigkeit und die Sickersäfte aus dem Mischbehälter und den Gärbehältern rekultiviert. D. h., diese Flüssigkeit wird mit aktiven Methanbakterien beimpft. Dazu wird aus dem Mischbebehälter durchgeimpftes und in Gärung befindliches Substrat abgezogen und mit der gesammelten Gärflüssigkeit und den Sickersäften im Impfkulturtank vermischt. Das mit Methanbakterien angereicherte Impfkonzentrat wird anschließend bis zum erneuten Einsatz im Mischbehälter unter Aufrechterhaltung der Prozesstemperatur zwischengelagert.
Neben dem gezielten Einsatz des Impfkonzentrates im Mischbehälter kann das Impfkonzentrat zur Unterstützung des Gärprozesses oder bei Störungen auch in den einzelnen Gärbehälter eingesetzt werden. Das im Impfkulturtank freigesetzte Methangas wird über den Kondensatabscheider und Wärmeaustauscher in den Gastank abgeleitet. Auftretende Flüssigkeitsverluste oder Flüssigkeitsüberschuss in oder aus der Biomasse werden entweder durch die Zuleitung von Frischwasser in den Impfkulturtank oder Ableitung der überschüssigen Gärflüssigkeit ausgeglichen.

Das vorgeschlagene Verfahren hat den Vorteil, dass eine kontinuierliche effektive Biogaserzeugung erreicht wird. Ein besonderer Vorteil ist, dass der biologische Prozess der Bildung aktiver Methanbakterien nicht unterbrochen wird, in dem vor der Beschickung der Anlage mit frischer Biomasse bis zum Abzug der Gärreste ein luftdichter Abschluss gewährleistet ist. Ein positiver Vorteil besteht auch darin, dass die erforderliche Prozessenergie in vollem Umfang aus dem Fermentationsprozess gewonnen wird und der Einsatz von Fremdenergie maximal in der Anfahrphase der Gesamtanlage notwendig ist. Positiv ist auch, dass keine Restgase in die freie Atmosphäre gelangen.

Das Verfahren bietet in vorteilhafter Weise und ohne anlagentechnische Anpassung die Möglichkeit, neben dem Einsatz mesophiler Bakterienkulturen den Fermentierprozess auch mit thermophilen Bakterienkulturen zu fahren. Damit besteht die Möglichkeit, dass neben dem Basissubstrat von Maissilage insbesondere auch Gras- oder Frischgetreide- und Sonnenblumensilage zur Biogaserzeugung effektiv eingesetzt werden kann.
Die Umstellung auf die jeweiligen Impfkulturen kann dabei während des laufenden Prozesses über die gezielte Aufzucht der Bakterien im Impfkulturtank erfolgen, was die Effizienz der Anlage grundsätzlich erhöht.

Die Einrichtung zur Durchführung des Verfahrens nach den Merkmalen in Anspruch 8 umfasst nachfolgend aufgeführte Baugruppen.

Zur Trockenfermatationsanlage gehören eine Entladestation mit Zerkleinerer für die einzutragenden Rohstoffe, ein geschlossener klimatisierter Mischbehälter zur Vorgärung, wenigstens drei geschlossene und klimatisierte Gärbehälter in verfahrensparalleler Anordnung, ein Pressschneckenseparator für die Gärreste, ein Kondensatabscheider und Wärmeaustauscher, ein Gastank, eine BHKW und ein mit dem Mischbehälter im Wirkungsverbund gekoppelter Impfkulturtank sowie ein Gärrestlager.
Ausstattungsgemäß umfasst die Anlage von der Aufnahme der Rohstoffe bis zum Abzug der Gärreste eine Transportkette, wobei die Fördermittel zwischen dem Mischbehälter und den Gärbehältern luft- und gasdicht abgeschlossen sind. Wie vorstehend angeführt, ist für die Aufnahme der zu vergärenden Rohstoffe eine Entladestation mit Aufnahmebehälter mit Bodenförderer, vorzugsweise einer Transportschnecke vorgesehen. Dem Aufnahmebehälter ist ein Zerkleinerer nachgeordnet.

Im Zerkleinerer wird das angelieferte, unterschiedlich strukturierte Rohmaterial zu einer Substratgröße mit einer durchschnittlichen Körnung von unter 10mm, vorzugsweise mit einer Körnung von 3mm zerkleinert. Als Zerkleinerer ist ein Häckselwerk vorgesehen. Dabei wird eine Substratdichte von ca. 0,80t/m³ angestrebt. Die nachfolgende Vergärung kann damit bei relativ geringem Einschluss von Luftsauerstoff effektiver und intensiver ablaufen. Im Endeffekt wird eine vollständige Umformung der Biomasse zu Biogas ermöglicht. Der Transport des zerkleinerten Substrates erfolgt anschließend mit einer stetig arbeitenden Förderschnecke oder einem Elevator in den Mischbehälter.

Im Mischbehälter findet die Vorgärung des Substrates statt. Der Mischbehälter besteht aus einer Stahl- oder Holzrahmenkonstruktion mit innenseitig bekleideten Mineralgussplatten. In seiner Längsachse sind über dem Boden zwei gegenläufige Mischschnecken angeordnet, die während der Fermentation das gehäckselte Substrat im Mischbehälter mit wechselnden Drehrichtungen fortlaufend oder in Abhängigkeit des Fortschrittes der Gärung umwälzen.
An der Deckenplatte sind zum Benetzen des Subtrates mit dem Impfkonzentrat mehrere über die Fläche verteilte Sprühdüsen angeordnet.

Die Bodenplatte, Wände und Abdeckplatte des Mischbehälters bestehen aus dem hochfesten, chemisch beständigen Werkstoff Mineralguss mit einem natürlichen mineralischen Füll- und Zuschlagstoff von ca. 94% und einem Reaktionsharz (PMMA) auf Acrylbasis als Bindemittel. Diese Platten sind mit einer korrosionsbeständigen Stahl- oder GFK-Bewehrung ausgestattet und haben eine Plattenstärke von 20 bis 30 mm. Neben der chemischen Beständigkeit besitzen sie eine hohe Abriebfestigkeit und zeichnen sich gegenüber Beton durch eine vierfach höhere Biegezugfestigkeit aus.
Die Bodenplatte und Wände sind als beheizbare Wärmeplatten ausgebildet, indem auf der Fermenterseite unmittelbar hinter der Mineralgussaußenhaut in einem Abstand von 5 bis 10 mm in die Wärmeplatten ein Heizrohrregister eingegossen ist. Damit wird eine effektive Wärmeübertragung gewährleistet und eine kontrollfähige Prozesssteuerung bezüglich der Einhaltung einer optimalen Prozesstemperatur von 36 bis 38°C bzw. 50 bis 57°C ermöglicht.

Die Bodenplatte, Wände und Deckenplatte des Mischbehälters sind luft- und gasdicht miteinander zusammengefügt und außen wärmeisoliert. Eine Verkleidung, vorzugsweise aus Trapezblech, schützt vor Witterungseinflüssen und mechanischen Einwirkungen. Vorzugsweise sind die Eckverbindungen zwischen Bodenplatte und Wänden durch auflaminierte GFK-Matten luft-, gas- und druckdicht versiegelt. Die Wände in der Behälterlängsachse sind gegenüber der Bodenplatte trichterförmig angeordnet.

Die für die Prozessführung erforderliche Wärmeenergie wird aus den abgezogenen Gärresten, der Aggregatwärme des Pressschneckenseparators und dem abgeleiteten Biogas gewonnen. In der ersten Anfahrphase der Fermentation und bei Fehlenergie wird die benötigte Wärmeenergie hilfsweise vom separat hochgefahrenen BHKW abgegriffen. Zusammen wird die Wärmeenergie prozessabhängig über die Wärmeplatten in den einzelnen Stationen dem Fermentationsprozess zugeführt. Auf diese Weise wird im Mischbehälter das frisch eingetragene Substrat kurzfristig auf die gewünschte optimale Prozesstemperatur erwärmt und durch die gleichzeitig erfolgte allseitige Benetzung mit Impfkonzentrat zur Gärung gebracht.

Das in Folge erzeugte Biogas wird oberhalb der Substratmasse über eine Abzugsöffnung kontinuierlich aus dem Mischbehälter zum Gastank abgeleitet. Dem Mischbehälter sind mindestens drei parallel betriebene, geschlossene und auf Prozesswärme beheizte Gärbehälter nachgeordnet. Ihre konkrete Anzahl richtet sich nach dem Füllvolumen des Mischbehälters und dem Verhältnis der Verweilzeit des Substrates in Misch- und Gärbehälter. Der Gärbehälter ist nach dem gleichen Konstruktionprinzip wie der Mischbehälter ausgeführt. Sein Volumen ist der Austragsmasse des vorgegorenen Substrates aus dem Mischbehälter angepasst. Die Bodenplatte, Wände und Abdeckung des Gärbehälters bestehen ebenfalls aus gas-und flüssigkeitsdicht zusammengefügten Mineralgussplatten mit den vorgenannten Eigenschaften und Wandstärken. Die Bodenplatte und Wände sind mit Heizrohrregistern ausgestattete Wärmeplatten. Im Ständerrahmen sind die Wände trichterförmig eingebaut und geben am Grund einen Sammelkanal mit einer Förderschnecke frei. Für die Beschickung des Gärbehälters mit Substrat aus dem Mischbehälter sind Schneckenförderer und/oder Elevatoren vorgesehen. Die Substratverteilung zu den einzelnen Gärbehältern erfolgt mittels einzelner Steuerschieber und steuerbaren Fördermittelantrieben.
Die Förderschnecke im Sammelkanal, der Schneckenförderer oder der Elevator sind kinematisch dahingehend verblockt, dass das Substrat aus dem Gärbehälter über diese Transportkette in den Gärbehälter zurück befördert und damit umgeschichtet werden kann. Auf diese Weise wird die Verkrustung des Substrates und die Ausbildung von Gas- oder Trockennestern im Gärbehälter verhindert und einer Stagnation der Biogaserzeugung entgegenwirkt. Zusätzlich sind in der Abdeckung Sprühdüsen zum Eintrag von Impfkonzentrat vorgesehen. Diese können aktiviert werden wenn der Gärprozess stocken sollte.

Das Austragen der anfallenden Gärreste erfolgt mit der im Sammelkanal angeordneten Förderschnecke zum nachgeordneten Pressschneckenseparator. Der Separator ist mit einem Auffangtrichter ausgerüstet, der zugleich als Wärmeaustauscher ausgebildet ist. Dazu werden vorzugsweise die Mineralgussplatten mit Heizrohrregister verwendet, die im Umkehrschluss die mittransportierte Wärme aus den Gärresten abgreifen. Analog wird die beim Trocken pressen im Pressschneckenseparator erzeugte Wärme abgegriffen, die maßgeblich als Prozessenergie eingesetzt wird. Hinter dem Pressschneckenseparator befindet sich ein witterungsgeschütztes Gärrestelager. Für den Transport der ausgepressten Gärreste in das Lager sind Band- oder Kettenförderer vorgesehen. Von hier aus kann der individuelle Abtransport der Gärreste als Rohkompost mit der Qualität eines NP-Düngers (Stickstoff-Phosphatdünger) erfolgen.

Zur Versorgung des Mischbehälters mit Impfkonzentrat ist im Anlagenverbund ein separater, geschlossener und isolierter Impfkulturtank vorgesehen. Er ist beheizbar und vorzugsweise in drei Kammern mit Rührwerken unterteilt. Der Boden, die Wände und die Tankabdeckung sind aus Mineralguss, wobei der Boden und die Wände als Wärmeplatten mit Heizrohrregistern ausgeführt sind. Die einzelnen Kammern sind über Pumpen miteinander verbunden. Die erste Kammer dient als Sammelkammer für das ausgepresste Gärwasser und die eventuell anfallenden Sickersäfte. Die zweite Kammer ist die Faulkammer zur gezielten Aufzucht von Methanbakterien. Dazu ist diese Kammer mit dem Mischbehälter zur Entnahme von in Gärung befindlichem Substrat über eine Rohrleitung verbunden. Die dritte Kammer fungiert als Vorratsspeicher für das rekultvierte Impfkonzentrat.
Das bei der Bakterienvermehrung erzeugte Biogas wird über eine Sammelleitung zum Kondensatabscheider und Wärmeaustauscher und dem Gastank abgeführt.
Analog sind Mischbehälter und Gärbehälter zur Ableitung des erzeugten Biogases über diese Sammelleitung mit dem Kondensatabscheider und Wärmeaustauscher und dem Gastank verbunden. Am Gastank ist das BHKW und/oder eine stationäre Wirbelschichtfeuerung (SWSF) angeschlossen.

Im Kondensatabscheider und Wärmeaustauscher erfolgt der Wärmeabgriff der vom Biogas mittransportierten Wärmmeenergie, die prozessabhängig in die Heizrohrregister der einzelnen Arbeitsstationen (Misch-, Gärbehälter und Impfkulturtank) transportiert wird. Parallel dazu ist das BHKW an den Wärmekreislauf angeschlossen.

Im nachfolgenden wird der verfahrenstechnische Ablauf nochmals an einem Ausführungsbeispiel erläutert. Die Prinzipdarstellungen zeigen:
Fig. 1: das Prozessablaufschema
Fig. 2: den Mischbehälter im Querschnitt
Fig. 3: den Mischbehälter im Längsschnitt
Fig. 4: einen Gärbehälter im Längsschnitt
Fig. 5: den Impfkulturtank im Längsschnitt

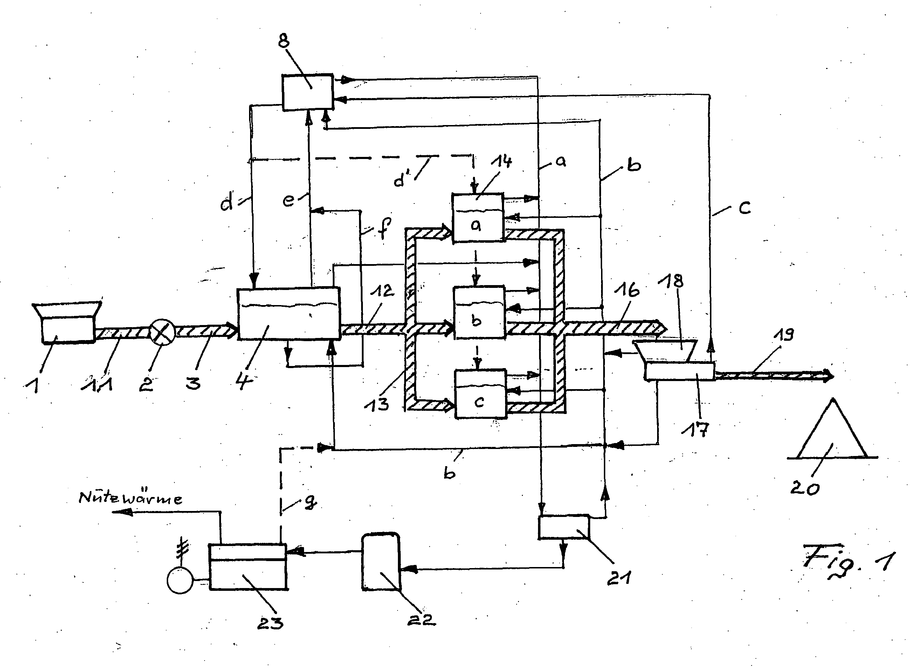

Entsprechend dem Prozessablaufschema nach Fig.1 wird die zur Trockenfermentation antransportierte Biorohmasse in den Aufnahmetrichter 1 entladen und mit einem Bodenförderer 1.1 zum Häcksler 2 gefördert. Hier wird es zu einem Substrat mit einer durchschnittlichen Körnung kleiner 10mm, vorzugsweise 3mm gehäckselt. Mittels der Förderschnecke 3 gelangt das Substrat in den geschlossenen und auf die Prozesstemperatur von 36 bis 38°C erwärmten Mischbehälter 4. Die Mischschnecken 6 bringen das eingetragene Substrat fortlaufend oder in Abhänigkeit der sich entwickelnden Vorgärung in Intervallen zum Umwälzen, wobei gleichzeitig das Substrat über die Sprühdüsen 7 mit Methanbakterien angereichertes Impfkonzentrat aus dem Impfkulturtank 8 besprüht wird. Damit wird das Substrat allseitig mit Impfkultur benetzt. Parallel dazu erfolgt der weitere Eintrag von Wärmeenergie über die Wand- und Bodenplatten. Damit wird zügig das gesamte Substrat auf das Niveau der Prozesstemperatur von 36 bis 38°C bzw. bei thermophilen Bakterien auf 50 bis 57°C erwärmt und diese Temperatur während der gesamten Gärung im Mischbehälter 4 aufrecht erhalten. Die anerobe Gärung des Substrates und Biogaserzeugung wird auf diese Weise bereits nach kurzer Verweildauer mit zunehmender Aktivität bewirkt.

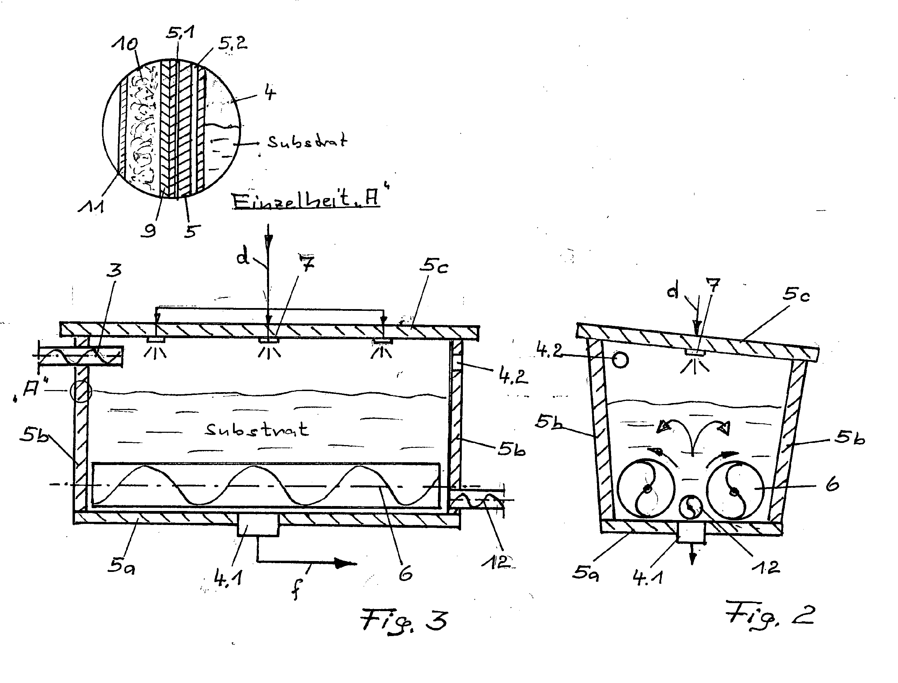

Fig. 2 und 3 zeigen den Mischbehälter 4 im Quer- und Längsschnitt. Er besteht allseitig aus chemisch beständigem hochfesten Mineralgussplatten 5 mit einem Wandquerschitt von 20 bis 30mm und einer korrosionsbeständigen Stahl- oder GFK-Bewehrung 5.1. Den Wandquerschnitt zeigt in einer Prizipdarstellung die Einzelheit "A". Die mit dem Substrat aktiv in Kontakt kommende Bodenplatte 5a und Wandplatten 5b sind als Wärmeplatten mit einem flächendeckenden Heizrohrregister 5.2 ausgeführt. Das Heizrohrregister 5.2 liegt vor der Bewehrung 5.1 unmittelbar (ca. 5mm) unter der Plattenoberfläche auf der Behälterinnenseite. Der Mischbehälter 4 ist aus der Bodenplatte 5a, Wandplatten 5b und Deckplatte 5c (ohne Heizrohrregister) flüssigkeits- und gasdicht zusammengefügt und wird von einem Ständerrahmen 9 (Stahl- oder Holzständerrahmen) abgestützt. Zusätzlich werden die Eckstöße von außen mit auflaminierten GFK-Laminatstreifen gesichert. Ebenso ist die Außenseite des Mischbehälters 4 mit Isoliermaterial 10 abgedeckt, die mittels Verblender 11 oder einer Trapezblechabdeckung vor Witterungseinflüsse oder Fremdeinwirkung gesichert wird.

In der Behälterlängsachse sind die Wandplatten 5b trichterförmig zur Bodenplatte 5a aufgestellt. Damit fällt das ständig umgewälzte Substrat immer auf die Mischschnecken 6 zurück. Zur Stabilität und Verschleißminderung sind diese Schnecken außerhalb des Mischbehälters 4 gelagert.

Zusätzlich ist in der Bodenplatte zur Aufnahme eventueller Sickersäfte ein Pumpensumpf 4.1 vorgesehen, der an den Impfkulturtank 8 angeschlossen ist.

Das Substrat wird nach Erreichen einer durchgreifenden aneroben Gärung nach ca. 8 bis 10 Tagen aus dem Mischbehälter 4 abgezogen und mit dem Schneckenförderer 12 und dem Elevator 13 direkt in den Gärbehälter 14 verbracht. Beide Fördermittel sind luft- und gasdicht gekapselt. Das erzeugte Biogas wird über die Abzugsöffnung 4.2 im Mischbehälter 4 zum Gastank 22 abgeleitet.

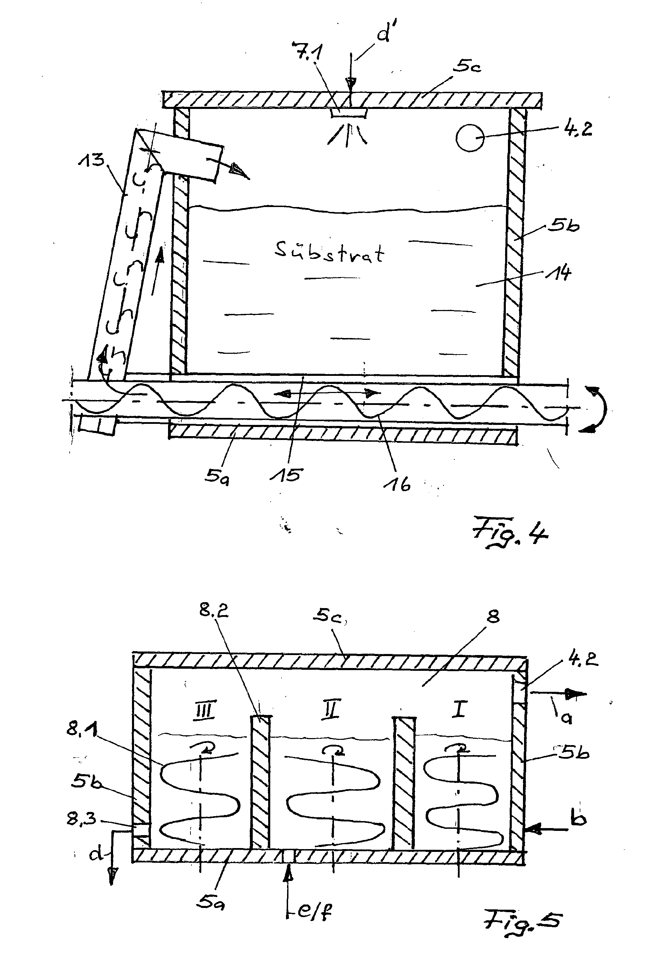

Das aus dem Mischbehälter 4 abgezogene Substrat wird von oben in den geschlossenen und auf Prozesstemperatur erwärmten Gärbehälter 14, wie Fig. 4 zeigt, ohne zu Verdichten eingefüllt. Im Gärbehälter 14 erfolgt die Abschlussgärung. Die Verweildauer beträgt ca. 25 Tage. Entsprechend der Vorgärphase im Mischbehälter 4 und der Abschlussgärung im Gärbehälter 14 sind zur Sicherung einer kontinuierlichen Biogasproduktion drei Gärbehälter 14a, 14b und 14c parallel zueinander aufgestellt.

Die konstruktive Grundstruktur der Gärbehälter 14a, b und c entspricht der des Mischbehälters 4. Die Wandplatten 5b sind im Gärbehälter 14 trichterartig eingebaut. In der Bodenplatte 5a ist in einem Sammelkanal 15 eine Förderschnecke 16 angeordnet. Die Boden-und Wandplatten 5a und 5b sind zum Eintrag der Prozesswärme mit Heizrohrregistern 5.2 ausgerüstet. Zum Umwälzen des Substrates ist die Förderschnecke 16 im Gärbehälter 14 mit dem Scheckenförderer 12 bzw. dem Elevator 13 wirkverbunden. Damit kann das Substrat zur Vermeidung einer Oberflächenverkrustung oder der Ausbildung von Gas- oder Trockennestern je nach Bedarf vorsorglich zwischen 3 bis 6 Tagen umgewälzt werden.
In der Deckenplatte 5a sind zusätzliche Sprühdüsen 7.1 eingesetzt. Sie werden aktiviert, wenn im Gärbehälter 14 eine Stockung des Gärprozesses eintreten sollte. In diesem Fall kann das Substrat zusätzlich mit dem Impfkonzentrat benetzt und die Stockung aufhoben werden.

Nach der Abschlussgärung werden die Gärreste, wie in Fig. 1 dargestellt, mit der Förderschnecke 16 entnommen und zum Pressschneckenseparator 17 gefördert. Die Gärreste gelangen zuvor in einen Aufnahmetrichter 18, der gleichzeitig als Wärmeaustauscher ausgebildet ist. Dementsprechend bestehen die Seitenwände aus Mineralgussplatten 5 mit eingegossenen Heizrohrregister 5.2 (hier nicht dargestellt). Über die Heizrohrregister wird im Umkehrschluss die von den Gärresten mittransportierte Wärmeenergie abgezogen. Gemeinsam mit der beim Auspressen der Gärrester anfallenden Wärme wird diese Wärmeenergie als Prozessenergie zurückgeführt. Aus dem Aufnahmetrichter gelangen die Gärreste zum Auspressen in den Pressschneckenseparator 17. Die ausgepresste Gärflüssigkeit wird anschließend in den Impfkulturtank 8 zurückgeleitet (Zuleitung c).
Die ausgepressten Gärreste werden dann mit einer Fördereinrichtung 19 (beispielsweise Förderband, Kratzförderer) in das witterungsgeschützte Gärrestelager 20 transportiert, von wo sie als Rohkompost zur individuellen Verwertung abgefahren werden können

Fig. 5 zeigt im Längsschnitt den geschlossenen und isolierten Impfkulturtank 8. Er ist in drei Kammern I, II und III unterteilt, die durch Förderpumpen miteinander verblockt sind. Die Kammern sind mit Rührwerken 8.1 angeordnet. Alle Plattenelemente bestehen aus Mineralguss. Die Bodenplatte 5a und die Wandplatten 5b sowie die Trennwände 8.2 sind mit Heizrohrregistern 5.2 ausgestattet. Über diese Elemente wird der Impfkulturtank 8, wie das Prozessablaufschema gem. Fig.1 zeigt, kontinuierlich mit der aus den Gärresten, der Separatorwärme und der abgegriffenen Wärmeenergie aus dem Biogas auf Prozesstemperatur erwärmt. Damit wird die in der Sammelkammer I des Impfkulturtankes 8 zurückgeführte Gärflüssigkeit beaufschlagt. Nach dem Erwärmen wird die relativ inaktive Gärflüssigkeit in die Faulkammer II umgesetzt und zum Aufbau eines neuen Impfkonzentrates mit aktivem Substrat und eventuell anfallenden Sickersäften (Zuleitung e und f) aus dem Mischbehälter 4 versetzt. Nach der Aktivierung und dem Anreichern mit Methanbakterien wir das gewonnene neue Impfkonzentrat in der Speicherkammer III zwischengespeichert um von dort nach Bedarf über die Zuleitung d und d' den Sprühdüsen 7 und 7.1 zum Benetzen des frischen Substrates im Mischbehälter 4 und/oder Gärbehälter 14 bereit gehalten. Der Abzug des Impfkonzentrates erfolgt über den Leitungsanschluss 8.3. Freigesetztes Biogas wird über die Abzugsöffnung 4.2 in die Sammelleitung a abgeführt.
Weiterhin ist im Prozessablaufschema nach Fig.1 die Anordnung des Kondensatabscheiders und Wärmeaustauschers 21 dargestellt. In diesem Aggregat wird das im Mischbehälter 4, den Gärbehältern 14a bis c und aus dem Impfkulturtank 8 gewonnene und über eine Sammelleitung a zugeführte Biogas entwässert und die mittransportierte Wärmeenergie für die Prozessführung abgezogen. Im Anschluss wird das Biogas zum Gastank 22 abgeleitet, wo es nach Bedarf an das BHKW 23 zur Verstromung und Wärmeerzeugung weitergeleitet wird.

Mit dem Kondensat aus dem Kondensatabscheider und dem Wärmeaustauscher 21 wird die aus dem Biogas freigesetzte Wärmeenergie im Verbund mit der erwärmten Kühlflüssigkeit vom Pressschneckseparator 17 als Prozesswärme zum Mischbehälter 4, den Gärbehältern 14a, b und c und dem Impfkulturtank 8 zurückgeführt (Flüssigkeitkreislauf b).
Fehlende Wärmeenergie wird vom BHKW 23 abgegeriffen. Dazu kann das BHKW 23 mit dem vorgenannten Flüssigkeitskreislauf zusätzlich verblockt (Zuleitung g) werden.
Die übrige im BHKW 23 gewonnene Wärmeenergie steht unabhängig von der Prozessenergie zum Abgriff für fremde Verbraucher zur Verfügung.

### Literatur:

/1/
Dir. u. Prof. Dr. P. Weiland, MSc.(agr.) A. Schattauer
"Biogaserzeugung durch Trockenvergärung von organischen Rückständen, Nebenprodukten und Abfällen aus der Landwirtschaft"
A1: Grundlagen der Trockenfermentation
A3: Stand der Technik
FAL-Institut für Technologie und Biosystemtechnik, Braunschweig,
Institut f. Energietechnik, Universität Rostock und
Institut f. Energietechnik u. Umwelt GmbH (IE), Leipzig, Juni 2006

### Bezugszeichenaufstellung

- 1: Aufnahmetrichter
- 1.1: Bodenförderer
- 2: Häcksler
- 3: Förderschnecke
- 4: Mischbehälter
- 4.1: Pumpensumpf
- 4.2: Abzugsöffnung
- 5: Mineralgussplatte
- 5a: Bodenplatte
- 5b: Wandplatte
- 5c: Deckplatte
- 5.1: Bewehrung
- 5.2: Heizrohrregister
- 6: Mischschnecke
- 7: Sprühdüse
- 7.1: Sprühdüse
- 8: Impfkulturtank
- 8.1: Rührwerk
- 8.2: Trennwand
- 8.3: Leitungsanschluss
- 9: Ständerrahmen
- 10: Isolierung
- 11: Verblender
- 12: Schneckenförder
- 13: Elevator
- 14: Gärbehälter
- 15: Sammelkanal
- 16: Förderschnecke
- 17: Pressschneckenseparator
- 18: Aufnahmetrichter
- 19: Fördereinrichtung
- 20: Gärrestlager
- 21: Kondensatabscheider und Wärmeaustauscher
- 22: Gastank
- 23: Blockheizkraftwerk (BHKW)

### Stoffleitungslegende:

- a): Biogas
- b): Kondensat und Kühlflüssigkeit
- c): Gärflüssigkeit
- d): Impfkonzentrat
- e): Impfsubstrat
- f): Sickersäfte
- g): BHKW-Wärme

## Patentansprüche

1. Verfahren zur diskontinuierlichen Trockenfermentation zur Erzeugung von Biogas aus nachwachsenden Rohstoffen (NaWaRo), vorzugsweise Maissilage sowie von Gras-, Frischgetreide- oder Sonnenblumensilage und organischen Rückständen, Nebenprodukten und Abfällen aus Haus- und Landwirtschaft mit vorrangig stichfester Konsistenz in einer Fermenteranlage bei gleichzeitigem Eintrag von Prozesswärme und dem Besprühen der frischen Biomasse mit einer mit Methanbakterien angereicherten Impfkultur, bei dem die Fermentierung in zwei Stufen erfolgt,
a) das frische Biomaterial vor der Fermentation zu einem Substrat mit einer durchschnittlichen Körnung von 10mm, vorzugsweise 3mm zerkleinert wird,
b) das Substrat in einen geschlossenen sauerstofffreien Mischbehälter (4) eingesetzt wird, bis dieser zu 2/3 mit Substrat befüllt ist,
c) der Mischbehälter (4) auf eine mesophile oder thermophile Prozesstemperatur von 32 bis 42°C oder 50 bis 57°C erwärmt ist, und die Wärme über die Boden- und Wandflächen eingetragen wird,
d) das Substrat fortlaufend oder in Abhängigkeit des Fortschrittes der Gärung umgewälzt wird, und das Umwälzen mit wechselnder Drehrichtung zweier gegenläufiger Mischschnecken (6) erfolgt,
e) das Substrat zur Gärung allseitig mit mesophilen oder thermophilen Methanbakterien angereichert wird,
f) die Verweilzeit des Substrates im Mischbehälter (4) zum Erreichen einer durchgreifenden Gärung 6 bis 10 Tage beträgt,
g) das produzierte Biogas oberhalb der Substratmasse aus den Mischbehälter (4) abgezogen wird, und wobei in einer zweiten Stufe,
h) mit Erreichen einer durchgreifenden Gärung das Substrat aus dem Mischbehälter (4) ohne Unterbrechung der Gärung unter Ausschluss von Sauerstoff in einen auf eine Prozesstemperatur von 36 bis 38°C oder 50 bis 57°C erwärmten Gärbehälter (14) umgesetzt wird,
i) das Substrat in Abständen von 3 bis 6 Tagen langsam umgewälzt wird, wobei die Verweilzeit zur Abschlussgärung des Substrates ca. 25 Tage beträgt, und das Biogas aus dem Gärbehälter (14) in den Gastank (22) abgeleitet wird, und wobei
j) schließlich die Gärreste aus den Gärbehälter (14) ausgebracht werden, und die Gärflüssigkeit bis zu 75% ausgepresst und in einen erwärmten Impfkulturtank (8) abgeleitet wird,
k) der Impfkulturtank (8) auf Prozesstemperatur erwärmt ist,
l) die gesammelte Gärflüssigkeit im Impfkulturtank (8) mit Methanbakterien angereichert und bis zur erneuten Verwendung gerührt und auf Vorrat gehalten wird,
m) die entwässerten Gärreste in einem witterungsgeschützten Gärrestlager (20) als Rohkompost zur weiteren Verwendung eingelagert werden,
n) die beim Auspressen anfallende Aggregatwärme als Prozessenergie genutzt wird,
o) wobei das Biogas in einem BHKW (23) verstromt und in Wärme umgesetzt wird.

2. Verfahren nach Anspruch 1, **gekennzeichnet dadurch, dass** zur Abschlussgärung im zeitlichen Wechsel mehr als ein Gärbehälter (14), vorzugsweise drei Gärbehälter (14a, 14b, 14c) mit gärendem Substrat aus dem Mischbehälter (4) befüllt werden.

3. Verfahren zur diskontinuierlichen Trockenfermentation nach Anspruch 1, **gekennzeichnet dadurch, dass** das Impfkonzentrat mit 5 % gärendem Substrat und anfallenden Sickersäften angereichert wird.

4. Verfahren zur diskontinuierlichen Trockenfermentation nach Anspruch 1, **gekennzeichnet dadurch, dass** sich die Anzahl der Gärbehälter (14a, b und c) gegenüber dem Mischbehälter (4) aus der Verweilzeit des Substrates im Gärbehälter (14) und der Vorgärzeit im Mischbehälter (4) im Verhältnis von 3:1 bestimmt.

5. Verfahren zur diskontinuierlichen Trockenfermentation nach Anspruch 1, dass in der Startphase oder bei Energiedefizit Prozesswärme vom anlagenzugehörigen BHKW (23) abgezogen wird.

6. Vorrichtung zur Durchführung des Verfahrens zur diskontinuierlichen Trockenfermentation nach Anspruch 1, wobei die Anlage folgende Baugruppen und Einzelelemente aufweist:
- eine Aufnahmevorrichtung für frisches Biorohmaterial
- eine Zerkleinerungsvorrichtung,
- einen klimatisch geschlossenen Mischbehälter (4),
- einen Impfkulturtank (8) für ein mit Methanbakterien angereichertes Impfkonzentrat,
- wenigstens drei klimatisch geschlossene Gärbehälter (14a, b u. c),
- einen Pressschneckenseparator (17),
- ein Kondensatabscheider und
- einen Wärmeaustauscher (21),
- einen Gastank (22),
- ein Blockheizkraftwerk (BHKW) (23)
- ein Gärrestlager (20) ,
- wobei die Aufnahmevorrichtung (1), die Zerkleinerungsvorrichtung (2), der Mischbehälter (4), die Gärbehälter (14a, b und c) und der Pressschneckenseparator (17) durch Fördervorrichtungen (1.1, 3, 12, 13, 16 und 19) wirkverbunden sind,
- wobei die Fördereinrichtungen (12, 13 und 16), gegenüber Luftsauerstoff abgeschirmt sind,
- ein klimatisch geschlossener Impfkulturtank (7) zur Beimpfung der Substratmasse im Mischbehälter (4) vorgesehen ist,
- wobei die Behälter (4, 14a,14b, 14c und 8) aus Mineralgussplatten (5) bestehen,
- wobei die Bodenplatte (5a) und die Wände (5b) mit Heizrohrregistern (5.2) bestückt sind,
- dass im Mischbehälter (4) zwei Mischschnecken (6) in Längsachse des Mischbehälters vorhanden sind,
- wobei im Gärbehälter (14) ein Sammelkanal (15) mit Förderschnecke (16) angeordnet ist,
- wobei im Impfkulturtank (8) Rührwerke (8.1) angeordnet sind,
- wobei zwischen dem Pressschneckenseparator (17) und Impfkulturtank (8) sowie dem Kondensatabscheider und Wärmeaustauscher (21) zur Aufnahme der Pressflüssigkeit und Kondensat eine Rohrverbindung (b) besteht,
- wobei der Mischbehälter (4), die Gärbehälter (14a, b und c) und der Impfkulturtank (8) zur Biogaserfassung über eine Sammelleitung (a) mit einem Kondensatabscheider und Wärmeaustauscher (21) verbunden sind,
- dass die Heizrohrregister (5.2) in den Mineralgussplatten (5) zum Transport der Wärmeenergie mit dem Kondensatabscheider und Wärmeaustauscher (21), dem Pressschneckenseparator (17) und Aufnahmetrichter (18) sowie hilfsweise mit dem BHKW (23) über einen Flüssigkeitskreislauf wirkverbunden sind.

7. Vorrichtung nach Anspruch 6, **gekennzeichnet dadurch, dass** der Aufnahmetrichter (18) des Pressschneckenseparators (17) aus Mineralguss und einem Heizrohrregister (5.2) besteht.

8. Vorrichtung nach Anspruch 6 und 7, **gekennzeichnet dadurch, dass** die Heizrohrregister (5.2) unmittelbar unter der Oberfläche der Mineralgussplatten (5a, 5b) angeordnet sind.

9. Vorrichtung nach Anspruch 6, **gekennzeichnet dadurch, dass** die Behälter (4, 14 und 8) isoliert und mit Verblender (11) abgedeckt sind.

10. Vorrichtung nach Anspruch 6, **gekennzeichnet dadurch, dass** der Impftank (8) in Kammern, vorzugsweise 3 Kammer (8a, b und c) unterteilt ist, die Trennwände (8.2) aus Mineralgussplatten (5) bestehen und mit Heizrohrregistern (4.2) ausgestattet sind und das in den Kammern Rührwerke (8.1) angeordnet sind und dass die Kammern (8a, b und c) miteinander durch Pumpen in Wirkverbund stehen.

11. Vorrichtung nach Anspruch 6, **gekennzeichnet dadurch, dass** die Förderschnecke (15) im Gärbehälter (14), der Schneckenförderer (12) und Elevator (13) zur Substratumschichtung im Gärbehälter (14) eine Transportkette bilden.

12. Vorrichtung nach Anspruch 6, **gekennzeichnet dadurch, dass** die Mischschnecken (6) außerhalb des Mischbehälters (4) gelagert sind.

13. Vorrichtung nach Anspruch 6, **gekennzeichnet dadurch, dass** die Behälter (4, 14 und 8) an ihren Verbindungsstellen der Mineralgussplatten (5) mit einem GFK-Laminat überlappt sind.

14. Vorrichtung nach Anspruch 6, dass als Zerkleinerungsvorrichtung ein Häcksler (2) vorgesehen ist.

15. Vorrichtung nach Anspruch 6, dass neben dem Blockheizkraftwerk (BHKW) (23) oder stattdessen eine stationäre Wirbelschichtfeuerung vorgesehen ist.
